# EUROPEAN PATENT SPECIFICATION

(11) **EP 4 633 552 B1**
(45) Date of publication and mention of the grant of the patent: **18.11.2026**
(21) Application number: 23836948.2
(22) Date of filing: 14.12.2023
(51) Int. Cl.: A61F 5/445

(54) **MEDICAL DEVICE FOR THE REALIZATION OF ILEOSTOMIES AND/OR JEJUNOSTOMIES**
MEDIZINISCHES GERÄT ZUR DURCHFÜHRUNG VON ILEOSTOMIEN UND/ODER JEJUNOSTOMIEN
DISPOSITIF MÉDICAL POUR LA RÉALISATION D'ILÉOSTOMIES ET/OU JÉJUNOSTOMIES

(30) Priority: 15.12.2022 IT 202200025761
(43) Date of publication of application: 22.10.2025
(73) Proprietor: EFG S.r.l., 00144 Roma (RM) (IT); SIDAM S.r.l., 41037 Mirandola (MO) (IT); Mariani, Enrico, 06039 Trevi (PG) (IT); Pagliacci, Emanuele, 06023 Gualdo Tadino (PG) (IT)
(72) Inventor: MARIANI, Enrico, 06039 Trevi (PG) (IT)
(74) Representative: Brunacci, Marco
(86) International application number: PCT/IB2023/062654
(87) International publication number: WO 2024/127284

(56) References cited:
- WO-A1-2013/088234
- WO-A1-2016/098064

## Description

### Technical Field

The present invention relates to a medical device for the realization of ileostomies and/or jejunostomies.

### Background Art

The scientific literature reports the need to practice protective ileostomy in cases of colorectal resection with ileocolic, colocolic, colorectal or coloanal anastomoses in order to reduce complications related to anastomotic dehiscences. For example, in the case of intestine cancer as a result of which surgical resection of the intestine itself has been performed with subsequent anastomosis, it is necessary to divert the flow of feces to prevent these from reaching the operated area, infecting it.

For this purpose, it is common practice to perform an ileostomy, which is to exteriorize the small intestine through the abdominal wall to divert the intestinal contents to an external container thus preventing it from progressing to the anastomosis.

Specifically, ileostomies can be of the temporary or permanent type depending on the pathology.

As can be easily guessed, temporary ileostomies are used in order to suspend the functions of a tract of the intestine for a certain period of time and to provide for the restoration of normal function of the intestine itself after such temporary suspension.

Permanent ileostomies, on the other hand, are used in the case of particular diseases and provide for permanent intestine detour.

The present disclosure mainly relates to the case of temporary ileostomies.

More specifically, currently, temporary ileostomies are performed by making a loop of the ileum, positioned upstream of the area to be safeguarded with respect to the direction of advancement of the enteric fluid, pass through an opening made in the abdominal wall (ostomy).

When the ileostomy has completed its protective function of the above-mentioned anastomoses, it can be removed by further surgery and then the intestinal continuity can be restored. The ileostomy closure procedure is performed on average about two months after the first surgery.

The pouching and subsequent closure of a temporary ileostomy have many drawbacks that are well described in the scientific literature.

The presence of the ileostomy involves not only high psychophysical discomfort for the patient who has to undergo various and invasive procedures under general anesthesia, but also long postoperative recovery times.

In addition, this procedure also results in high costs for health care facilities both in the management of the ileostomy (outpatient medical checkups for maintenance and treatment of any complications, disposable collection plaques and bags) and in the additional surgery required for the closure of the ileostomy (high-tech medical devices, use of operating theatres and related personnel).

In addition to this, it should be considered that the medical staff and equipment used for such surgeries are evidently taken away from the performance of other surgeries, a fact that causes the obvious extension of waiting times for other surgeries.

Therefore, the procedure for the realization of temporary ileostomies of the type described above proves to be as costly as it is in terms of healthcare logistics.

To solve at least part of the above drawbacks, the use of medical devices constructed in accordance with the teachings of patent WO2016098064A1 is known.

Within that document, a device is particularly described comprising a tubular element in which at least one passage channel is defined provided with a plurality of feces inlet openings, which can be inserted within the patient's intestine, and with at least one feces outlet opening, which can be positioned outside the intestine.

In detail, this device comprises obstructing means of an area of the intestine associated with the tubular element and positioned between the inlet opening and the outlet opening.

Specifically, the obstructing means are insertable within the intestine itself and are movable between a narrowed configuration and an enlarged configuration to enable their insertion within the intestine and to prevent the passage of feces through the aforementioned area of the intestine itself, respectively.

In the present case, the obstructing means substantially consist of a balloon pressure probe configured to inflate in the switch from the narrowed configuration to the enlarged configuration and, conversely, to deflate in the switch from the enlarged configuration to the narrowed configuration.

The probe is, therefore, able to expand radially and, in doing so, to come into contact with the walls of the intestine, thus obstructing the passage of feces to the aforementioned area of the latter.

In this regard, it is important to explain that the medical device allows remedying the problems complained of so far and, in particular, significantly limiting invasive surgery on the patient for the application and removal of an ileostomy.

In fact, it is applied during surgery aimed at treating intestinal pathology and its removal is done on an outpatient basis, without the use of any anesthesia.

In particular, in the case of surgeries performed laparoscopically, it is also possible to take advantage of the same holes used for the surgery to apply the device concerned, thus minimizing, in this way, surgery on the patient.

This fact results in a significant reduction in recovery time compared with the use of prior arts, as the removal of the ileostomy takes place in a matter of moments and the patient, in addition to not having to undergo further surgery, does not even have to suffer the consequences of a new total anesthesia.

While achieving the aforementioned benefits, the medical device described in patent document WO2016098064A1 is, nevertheless, amenable to refinements aimed at further improving its effectiveness and convenience of use.

First of all, in the practice of use, it has been found that the high pressure within the intestine can cause the probe to be displaced on the tubular element during the explantation of the device, resulting in hindering the performance of the explantation itself as well as undermining the proper function of the device in its subsequent uses.

In addition, it is important to point out that the probe and the tubular element have rather different thicknesses at the point of transition from the former to the latter; due to this fact, the transition from the probe to the tubular element does not occur, during the insertion of the device into the patient's intestine, in a smooth and gradual manner, but rather abruptly and quickly.

It is easy to appreciate how, for this reason, the aforementioned insertion can be the cause of considerable physical and psychological discomfort for the patient, leading, as a direct result, to complications in the performance of the operation as well as a potential slowdown in recovery therefrom.

### Description of the Invention

The main aim of the present invention is to devise a medical device for the realization of ileostomies and/or jejunostomies which is reliable, functional and easily explanted from the patient's intestines.

Within the aforementioned main aim, one object of the present invention is to devise a medical device for the realization of ileostomies and/or jejunostomies, the use of which results in a significant reduction in the physical and psychological discomfort suffered by the patient.

Another object of the present invention is to devise a medical device for the realization of ileostomies and/or jejunostomies which, while maintaining good probe flexibility, is provided with a rigid and strong structure.

Another object of the present invention is to devise a medical device for the realization of ileostomies and/or jejunostomies which allows the aforementioned drawbacks of the prior art to be overcome within the framework of a simple, rational, easy and effective to use as well as cost-effective solution.

The objects explained above are achieved by this medical device for the realization of ileostomies and/or jejunostomies having the characteristics of claim 1.

### Brief Description of the Drawings

Other characteristics and advantages of the present invention will become more apparent from the description of a preferred, but not exclusive, embodiment of a medical device for the realization of ileostomies and/or jejunostomies, illustrated by way of an indicative, yet non-limiting example in the accompanying tables of drawings in which:
Figure 1 is an axonometric, overall view of the device according to the invention;
Figure 2 is a detailed view, in partial transparency, of Figure 1;
Figure 3 is a cross-sectional view of the device according to the invention;
Figure 4 is a longitudinal sectional view of the device according to the invention;
Figure 5 and Figure 6 show the operation of the device according to the invention in accordance with a first embodiment;
Figure 7 shows a second embodiment of the device according to the invention.

### Embodiments of the Invention

With particular reference to these figures, reference numeral 1 globally denotes a medical device for the realization of ileostomies and/or jejunostomies.

The medical device 1 for the realization of ileostomies and/or jejunostomies first comprises at least one tubular element 2 comprising, in turn, at least one passage channel 3 provided with a plurality of inlet openings 4 of feces, insertable within the intestine I of a patient, and with at least one outlet opening 5 of feces, positionable outside the intestine I.

The tubular element 2 is made of a radio-opaque material, e.g. silicone or polyurethane.

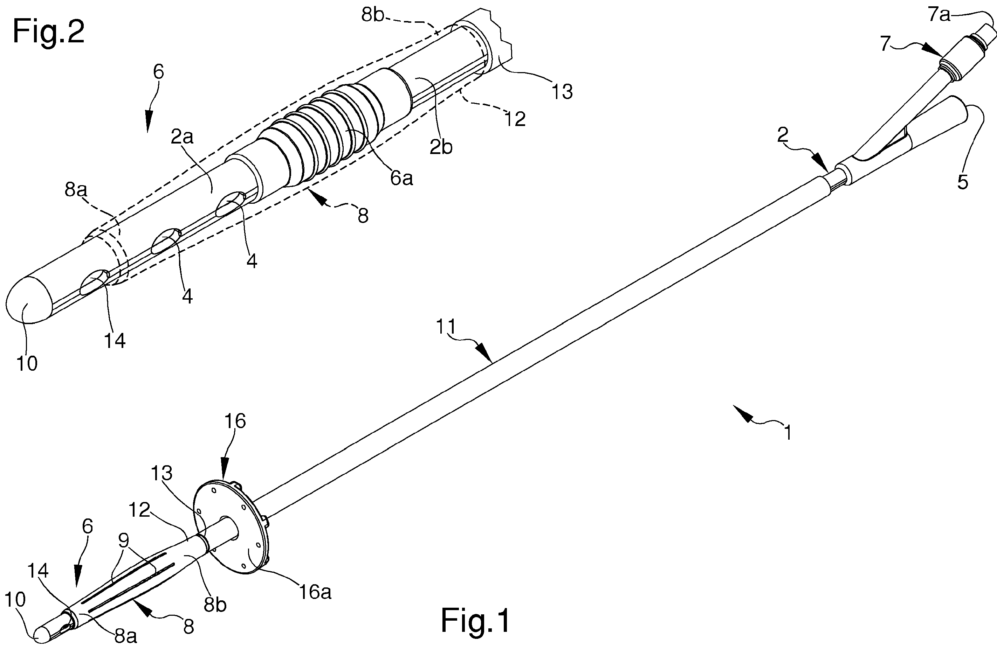

More particularly, as visible in Figure 2, the tubular element 2 comprises at least a first section 2a on which the inlet openings 4 are defined and at least a second section 2b.

Specifically, on the second section 2b, the outlet opening 5 is obtained (Figure 1). The first section 2a is insertable within the patient's intestine I through an ostomy obtained, e.g., on the patient's abdomen A in order to perform a laparoscopic operation, such as surgery to resect a section of the ileum.

Advantageously, the inlet openings 4 are defined at the lateral surface of the first section 2a.

The inlet openings 4 are distributed in such a way as to allow drainage of fluids introduced into the passage channel 3 along the substantial entirety of the lateral surface of the first section 2a.

In this regard, at least two of the inlet openings 4 are arranged along the longitudinal development of the first section 2a.

Specifically, all the inlet openings 4 are arranged along the longitudinal development of the first section 2a.

Specifically, the inlet openings 4 are aligned to define two mutually parallel rows obtained on the first section 2a in diametrically opposite positions to each other. Preferably, the inlet openings 4 have a substantially elliptical conformation.

In more detail, the major axis of the inlet openings 4 is arranged substantially parallel to the longitudinal development of the first section 2a.

The inlet openings 4 are of a size such as to allow the outflow of feces within the passage channel 3 and, at the same time, to prevent the tubular element 2 from weakening.

In fact, the elliptical shape of the inlet openings 4 allows for the greatest possible outflow with equal weakening of the first section 2a.

In this regard, that is, in order to allow for an adequate number of inlet openings 4 having the aforementioned dimensions, the length of the first section 2a is substantially of between 8 cm and 10 cm.

The second section 2b is, on the other hand, intended to be arranged outside of the intestine I and the outlet opening 5, arranged at its axial end, is associable with a feces collection container not shown in the figures.

Preferably, the second section 2b has a length substantially equal to 40 cm.

Having described the two sections 2a, 2b of the tubular element 2, the device 1 comprises obstructing means 6 of at least one zone Z of the intestine I associated with the tubular element itself.

Specifically, the obstructing means 6 are insertable within the intestine I and are movable between a narrowed configuration and an enlarged configuration to allow the insertion thereof within the intestine I and to prevent the passage of feces through the zone Z, respectively.

As visible in Figure 2, the obstructing means 6 are positioned between the first section 2a and the second section 2b.

The obstructing means 6 are adapted to isolate the part of the intestine I arranged downstream of the zone Z with respect to the direction of feces advancement.

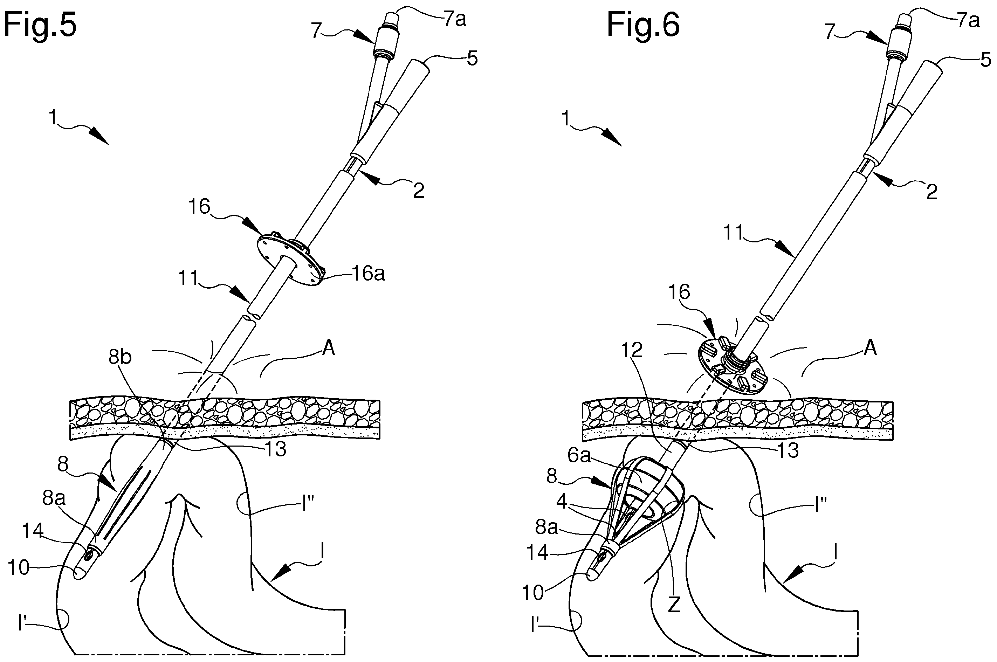

In other words, the obstructing means 6 allow the intestine I to be divided into two portions, shown by way of example in Figures 5 and 6, of which a first portion I' and a second portion I" arranged downstream and upstream respectively of the obstructing means themselves with respect to the direction of feces advancement. Thus, the first portion I' is in communication with the patient's stomach, while the second portion I", comprising the portion to be safeguarded, corresponds to the part whose functions are to be suspended, e.g. that in which an anastomosis has been made.

The inlet openings 4 and, therefore, also the first section 2a are intended to be housed into the first portion I' of the intestine I, i.e., upstream of the obstructing means 6 always with respect to the direction of feces advancement, so as to allow the interception of excretions from the digestive tract.

In this sense, the obstructing means 6 are configured to prevent the passage of feces from the first portion I' to the second portion I", that is, as anticipated, through the zone Z.

Preferably, the obstructing means 6 comprise at least one inflatable balloon pressure probe 6a.

That probe 6a is arranged externally to the tubular element 2 and is substantially deflated in the narrowed configuration (Figure 5) and inflated in the enlarged configuration (Figure 6).

In accordance with a first embodiment visible in the aforementioned Figures 5 and 6, the obstructing means 6 comprise a single probe 6a.

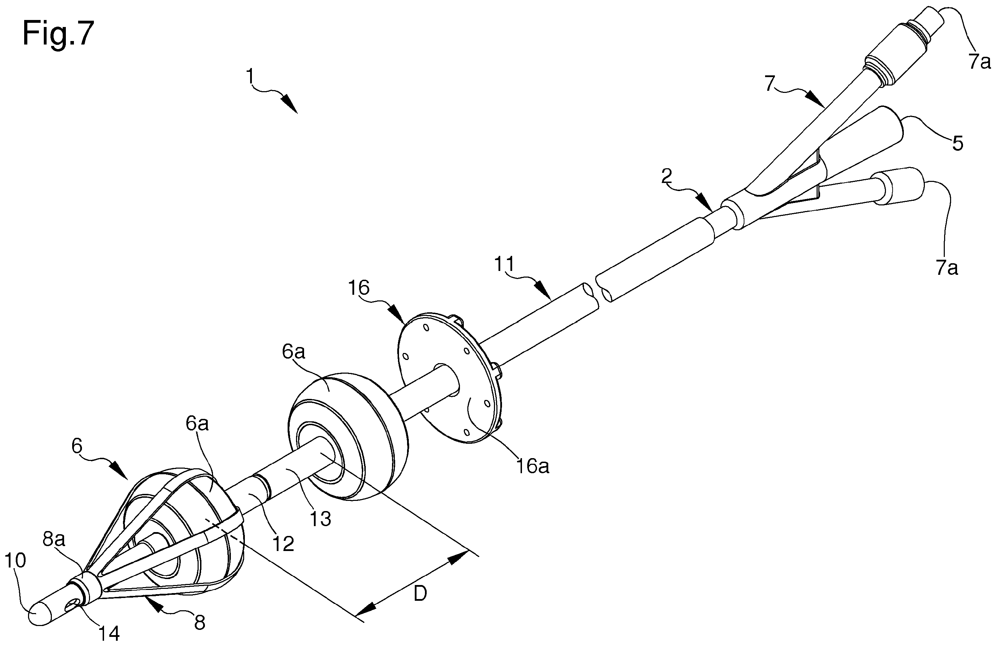

In accordance with a second embodiment, shown in Figure 7, the obstructing means 6 comprise two probes 6a arranged, along the device 1, at a reference distance D from each other.

Specifically, the reference distance D is of between 5 cm and 25 cm, better still of between 10 cm and 20 cm, preferably of 15 cm.

It should be noted, in this regard, how the particular expedient of providing an additional probe 6a compared with the first embodiment allows for the definition of an anchoring point within the patient's intestine I, with the advantageous result of increasing the stability of the device 1 and, consequently, its practicality and ease of use.

The possibility cannot, however, be ruled out of realizing the device 1 in accordance with further embodiments wherein an even greater number of probes 6a, such as three, are provided.

In all cases, the device 1 comprises activation means 7 of the probe 6a.

The activation means 7 comprise at least one lumen 7a for air inlet and outlet communicating with the probe 6a through a related conduit associated with the tubular element 2.

The conduit that set the lumen 7a in communication with the probe 6a is separate from the passage channel 3.

In the first embodiment, the activation means 7 comprise a single lumen 7a communicating with the single probe 6a (Figure 5 and Figure 6).

Regarding the second embodiment, the activation means 7 comprise two lumens 7a each of which is communicating with a respective probe 6a (Figure 7).

The activation means 7 also comprise a check valve, not visible in detail in the figures, adapted to prevent the accidental escape of air contained in the probe 6a and controllable by a user to allow air to flow outwards.

Advantageously, the obstructing means 6 comprise a plurality of elastically deformable tensioning elements 8 associated with at least the first section 2a to prevent it from bending.

Specifically, the tensioning elements 8 externally cover the first section 2a and the obstructing means 6.

Specifically, the tensioning elements 8 have substantially filiform conformation and are spaced apart from each other to define a plurality of through gaps 9 adapted to allows the passage of the intestinal contents to the inlet openings 4.

Advantageously, the tensioning elements 8 are locked together to define a single body piece.

Specifically, the tensioning elements 8 are locked together where their respective ends are located.

In this regard, it is important to specify that the tensioning elements 8 just described have a first end 8a associated with the first section 2a in the proximity of the free end 10 of the tubular element 2 (i.e., the end of the latter arranged, in use, within the patient's intestine I, i.e., the one arranged adjacent to the first section 2a, the latter being positioned between the free end 10 and the obstructing means 6) and a second end 8b opposite the first end 8a.

In more detail, in the narrowed configuration these tensioning elements 8 are in a home configuration wherein they substantially adhere to the tubular element 2 (Figure 1, Figure 4 and Figure 5).

Again, in the enlarged configuration these tensioning elements 8 are in an active configuration wherein they are spaced apart from the profile of the tubular element 2 (Figure 6).

Specifically, in the active configuration, the tensioning elements 8 take on a divergent conformation from the free end 10 towards the probe 6a.

This means that, in the active configuration, the tensioning elements 8 are tensioned by the probe 6a so as to impart stiffness to the first section 2a in order to prevent the latter from bending on itself thus obstructing the inlet openings 4. For example, the tensioning elements 8 are made of a material suitable for medical use such as polyurethane or silicone.

According to the invention, the device 1 comprises at least one sheath member 11 associated with the tubular element 2, which is arranged in abutment against the obstructing means 6 and adapted to counteract the relative sliding between the obstructing means 6 and the tubular element 2.

In particular, the sheath member 11 is preferably made of at least partly of at least one plastic material.

More specifically, the sheath member 11 is made at least partly of silicone.

Conveniently, the sheath member 11 is made entirely of silicone.

Indeed, such a material makes it possible to create a perfectly biocompatible, healthy and hygienic sheath member 11 whose use on humans is, therefore, absolutely safe.

In terms of its geometry, the sheath member 11 has a substantially tubular shape. In particular, the diameter of the sheath member 11 is larger than that of the tubular element 2.

In particular, the diameter of the sheath member 11 is such that it can be fitted substantially to size on the tubular element 2.

Conveniently, the obstructing means 6 comprise at least one contact portion 12 abutting, in use, against the sheath member 11.

As visible in the figures, the contact portion 12 has a substantially cylindrical conformation.

In this regard, it is important to specify that the contact portion 12 is associated with the tensioning elements 8 just described to define a single body piece with them (see Figure 2 in this regard).

Specifically, the tensioning elements 8 are associated with the contact portion 12 through their second end 8b.

Therefore, the contact portion 12 slides along the tubular element 2 as a result of the passage of the tensioning elements 8 from the home configuration to the active one or vice versa, or as a result of the passage of the obstructing means 6 from the narrowed configuration to the enlarged configuration or vice versa.

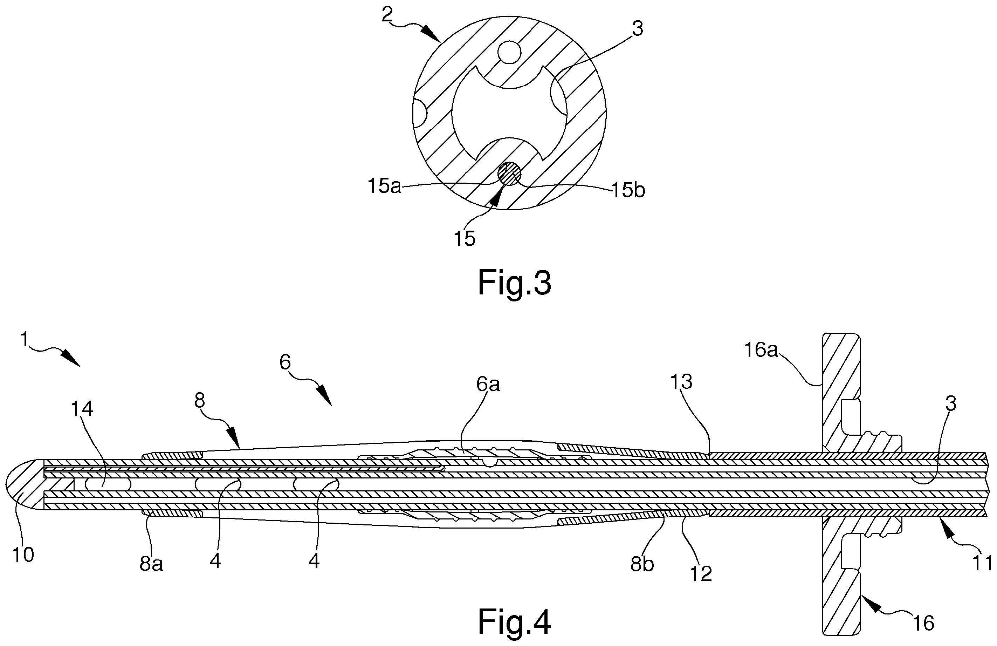

In the present case, as clearly visible in Figure 4, the contact portion 12 and the sheath member 11 have, where their contact area is located, substantially coinciding transverse dimensions to define a seamless outer joining surface 13.

To assert this is to say that the contact portion 12 and the sheath member 11 have conformations such that a straight line tangent to their outer surfaces does not encounter, in the transition between the two, any kind of geometric discontinuity. In practice, the contact portion 12 and the sheath member 11 are mutually joined so as to collectively define a substantially uninterrupted perimeter surface.

It is important to explain that what has been described so far on the sheath member 11 allows, in the context of a technical solution that is easy to implement and of limited cost, remedying the drawbacks of the state of the art previously complained of.

First of all, the fact that the sheath member 11 abuts against the obstructing means 6, thus preventing their relevant sliding with respect to the tubular element 2, averts the risk of the probe 6a slipping out of the tubular element itself when explanting the device 1.

This fact makes it possible, therefore, to greatly promote the operation of explanting the device 1 as well as to ensure the proper function of the latter in its subsequent uses.

In addition, the unique conformation of the sheath member 11 and, in particular, the substantially uninterrupted outer joining surface 13 between the member and the obstructing means 6 allows for a smooth and gradual insertion of the device 1 into the patient's intestine I.

It is easy to appreciate how, for this reason, the device 1 can be profitably used to perform ileostomies and/or jejunostomies without causing any physical and psychological discomfort on the patient, leading, as a direct result, to a simplification of the operation and, therefore, of recovery from the same.

Having specified these advantages, it is added that, favorably, the tubular element 2 comprises drainage means 14, clearly visible in Figures 2 and 4, adapted to promote the drainage of fluids present in the passage channel 3 outside the latter. In this sense, the drainage means 14 operate in conjunction with the inlet openings 4 to allow the fluids present in the passage channel 3 to be conveyed outside the latter even more effectively.

In this regard, it is worth noting that the use of the drainage means 14, which is useful for any type of fluid, is particularly convenient for evacuating from the passage channel 3 any lubricating fluids introduced therein for the purpose of promoting the insertion and/or extraction of the device 1 into/from the patient's intestine I.

Going into more detail, the drainage means 14 comprise at least one drainage hole 14 passable through by the fluids to be drained and obtained on at least one of either the first section 2a or the free end 10.

Preferably, the drainage hole 14 is obtained on the first section 2a.

Specifically, the drainage hole 14 is located, on the first section 2a, between the free end 10 and the inlet openings 4 (Figure 2).

The location of the drainage hole 14 is particularly convenient because it allows lowering the risk of the same being obstructed by feces introduced through the inlet openings 4.

As clearly visible in Figures 2 and 4, the drainage hole 14 has a substantially elliptical conformation.

In this sense, the drainage hole 14 is sized substantially similarly to the inlet openings 4.

It is specified that, conveniently, the drainage means 14 comprise two drainage holes 14 obtained on the first section 2a in diametrically opposite positions to each other.

Specifically, the two drainage holes 14 are placed at equal distances from the free end 10 of the tubular element 2 and therefore make a through hole on the first section 2a having transverse development to the latter.

As visible in the cross section of the tubular element 2 shown in Figure 3, the device 1 comprises stiffening means 15 adapted to counteract the bending of the tubular element itself.

Specifically, the stiffening means 15 comprise:
- at least one containment channel 15a cut out longitudinally through the tubular element 2; and
- at least one stiffening element 15b inserted within the containment channel 15a.

Thus, the stiffening means 15 make it possible to obtain a device 1 which, while retaining good flexibility of the probe 6a, is provided with a sufficiently rigid and strong structure that gives it high reliability and effectiveness in use.

In this sense, the stiffening means 15 operate in conjunction with the sheath member 11 in counteracting the relevant displacement between the obstructing means 6 and the tubular element 2 with additional strength, thus increasing even more the advantages just above concerning the sheath member itself.

In this regard, it is important to specify that, preferably, the containment channel 15a and the stiffening element 15b have a longitudinal extension substantially coinciding with that of the tubular element 2.

In other words, the containment channel 15a extends substantially by the entire longitudinal development of the tubular element 2 and has a length substantially coinciding with that of the stiffening element 15b.

The fact of providing a stiffening element 15b fully inserted into the tubular element 2 by the entire length of the latter makes it possible to increase the bending stiffness of the tubular element itself and, therefore, to significantly strengthen the structure of the latter.

In the preferred embodiment, the stiffening element 15b has filiform conformation and is made at least partly of a metallic material.

In this regard, the stiffening element 15b has thickness of less than 1 mm.

Specifically, the stiffening element 15b has thickness of between 0.3 mm and 0.9 mm, better still of between 0.4 mm and 0.8 mm, preferably of between 0.5 mm and 0.7 mm.

In this sense, the containment channel 15a has similar transverse dimensions to those of the stiffening element 15b just provided so as to allow the latter to be fitted to size within it.

Conveniently, the device 1 comprises retaining means 16 associated with the sheath member 11 at a predefined distance from the obstructing means 6 and intended, in use, to be placed in abutment against the patient's body.

In this case, the retaining means 16 are configured to operate in conjunction with the patient's abdomen A to block the position of the obstructing means 6 with respect to the patient's body (see Figure 6 in this regard).

In more detail, the retaining means 16 comprise at least one retaining element 16 drilled and fitted on the sheath member 11 in a sliding manner.

Specifically, the retaining element 16 defines a contact surface 16a intended to rest against the patient's body.

More specifically, the contact surface 16a is substantially flattened in shape.

In the embodiment shown in the figures, the retaining element 16 is of the type of a disk provided with a central through hole traversed by the tubular element 2 and by the sheath member 11 fitted on the latter.

In practice, the central hole has a diameter substantially coinciding with the cross-sectional dimension of the sheath member 11.

Preferably, the retaining element 16 is made of a material with a high coefficient of friction (e.g., silicone), so that it is difficult for it to slide with respect to the tubular element 2.

In use, therefore, the retaining element 16 is made to slide over the sheath member 11 until its contact surface 16a is brought to rest against the patient's abdomen A (compare Figure 5 and Figure 6).

Different embodiments of the retaining element 16 not depicted in the attached figures cannot however be ruled out and provided, e.g., with guiding means of the sheath member 11.

Such guiding means are, e.g., made up of a housing seat of one portion of the sheath member 11, which develops along a direction substantially inclined with respect to the axis of the through hole.

More particularly, the housing seat develops along a direction substantially parallel to the contact surface 16a of the retaining element 16.

In a further embodiment, the retaining element 16 may also comprise fixing means consisting of, e.g., one or more through holes associable with the patient's body, e.g. by means of suture.

Regardless of the embodiment chosen, the presence of the retaining means 16 clearly allows the position of the obstructing means 6 to be blocked into the specific zone Z of the intestine I with precision and simplicity, greatly facilitating the performance of the operation on the patient and thus promoting the patient's psychological and physical well-being.

The operation of this invention is as follows.

It is assumed that an ostomy has already been performed on the patient's abdomen A, e.g., to perform laparoscopic surgery on the patient's intestine, or specifically to perform ileostomy and/or jejunostomy.

At the end of the surgical operation involving the intestine, the tubular element 2 is made to pass through the aforementioned ostomy, so that its first section 2a is inserted inside the intestine I, where there is the zone Z located upstream of the operated part with respect to the direction of feces advancement.

The tubular element 2 is pushed within the intestine I so that the inlet openings 4 are directed towards the patient's stomach.

Eventually, if lubricating liquids are to be inserted into the passage channel 3 to facilitate the insertion of the device 1, they can be effectively evacuated from the device itself by their transiting through the inlet openings 4 and the drainage hole 14.

The obstructing means 6 are also introduced within the intestine I and, with them, the contact portion 12 and the part of the sheath member 11.

As discussed above, the presence of the outer joining surface 13 between the contact portion 12 and the sheath member 11 allows for a smooth transition from the second to the first during the insertion of the device 1 into the ostomy, thus lowering the risk of psychophysical discomfort by the patient.

Obviously, during the insertion of the tubular element 2 within the intestine I, the probe 6a is in the narrowed configuration in order to promote the passage thereof through the ostomy.

It follows, therefore, that during this phase, the tensioning elements 8 are also in the home configuration.

The tubular element 2 is then inserted in the manner just described and the above surgical operation completed, the part of the intestine affected by the operation is isolated by performing the ileostomy and/or jejunostomy.

More particularly, the probe 6a is brought into the enlarged configuration by blowing air through the lumen 7a.

In this way, the probe 6a swells and, as it enlarges, goes to occupy the entire section of the zone Z in which it is inserted, thus obstructing it.

The probe 6a is deformable and, when in the enlarged configuration, therefore, it conforms to the inner walls of the intestine I.

Following the inflation of the probe 6a, the tensioning elements 8 also move from their home configuration to the active configuration, in which they are tensioned as a result of the enlargement of the probe itself so as to impart stiffness to the first section 2a.

Contributing to this fact are the stiffening means 15, which operate synergistically in conjunction with the tensioning elements 8 in keeping the first section 2a substantially extended.

As anticipated above, the first section 2a is arranged, in use, upstream of the obstructing means 6, that is, along the first portion I' of the intestine I.

Appropriately, by exerting a traction force on the second section 2b which has remained outside of the intestine I and protruding from the patient's body, the probe 6a is brought to the ostomy.

At this point, the retaining means 16 are brought close to the patient's body by making them slide along the second section 2b and by bringing the contact surface 16a to rest against the patient's skin.

In this way, the tubular element 2 is blocked with respect to the patient's body as the retaining means 16 and the probe 6a are arranged on the opposite sides of the ostomy.

Excretions arriving from the patient's stomach and passing through the first portion I' of the intestine I are then intercepted by the probe 6a, which prevents the advancement thereof into the second portion I" and, therefore, enter the passage channel 3 through the inlet openings 4 defined along the first section 2a and arranged so as to substantially cover all of its lateral wall.

The feces then pass through the passage channel 3 until they reach the outlet opening 5 through which they are conveyed to the appropriate collection container.

To remove the ileostomy and/or jejunostomy, it is sufficient to deflate the probe 6a by operating on the valve placed in the proximity of the lumen 7a and to pull the tubular element 2 out through the ostomy.

Similarly to the above, if lubricating liquids are planned to be inserted into the passage channel 3 to facilitate the extraction of the device 1, the latter are effectively evacuated through the inlet openings 4 and the drainage means 14.

Once the tubular element 2 has been removed, the intestine I is again unimpeded and can thus resume its normal functioning, whereby the excretions emitted from the stomach pass through the now free zone Z and travel through the second portion I" of the intestine itself.

The ostomy obtained on the patient's abdomen A is then properly medicated and protected until it is completely healed.

It has in practice been ascertained that the described invention achieves the intended objects.

In particular, the fact is emphasized that the special expedient of providing a sheath member arranged against the obstructing means allows the probe to be held firmly in place on the tubular element during the use of the device and, in particular, during the removal of the same from the patient's intestine.

This fact makes it possible to greatly simplify the conduct of the aforementioned explantation operation and to drastically increase the reliability of using the device compared with the aforementioned state of the art.

Finally, it is specified that providing a sheath member defining with the obstructing means a seamless outer joining surface allows for a smooth and gentle transition from the obstructing means themselves to the tubular element during the insertion of the device into the patient's body, resulting in reduced physical pain and psychological stress experienced by the patient as well as facilitating the postoperative recovery of the latter.

## Claims

1. Medical device (1) for the realization of ileostomies and/or jejunostomies, comprising:
- at least one tubular element (2) comprising at least one passage channel (3) provided with a plurality of inlet openings (4) of feces, insertable within the intestine (I) of a patient, and with at least one outlet opening (5) of feces, positionable outside the intestine (I), wherein said tubular element (2) comprises at least a first section (2a), on which said inlet openings (4) are defined, and at least a second section (2b), on which said outlet opening (5) is defined;
- obstructing means (6) of at least one zone (Z) of the intestine (I) associated with said tubular element (2), insertable within the intestine (I) and movable between a narrowed configuration and an enlarged configuration to allow the insertion thereof within the intestine (I) and to prevent the passage of feces through said zone (Z) respectively, said obstructing means (6) being located between said first section (2a) and said second section (2b);
**characterized by** the fact that it comprises at least one sheath member (11) associated with said tubular element (2), arranged in abutment against said obstructing means (6) and adapted to counteract the relevant sliding between said obstructing means (6) and said tubular element (2).

2. Device (1) according to claim 1, **characterized by** the fact that said obstructing means (6) comprise at least one contact portion (12) abutting, in use, against said sheath member (11), said contact portion (12) and said sheath member (11) having, where their contact area is located, substantially coinciding transverse dimensions to define a seamless outer joining surface (13).

3. Device (1) according to one or more of the preceding claims, **characterized by** the fact that said sheath member (11) is made at least partly of at least one plastic material.

4. Device (1) according to claim 3, **characterized by** the fact that said sheath member (11) is made at least partly of silicone.

5. Device (1) according to one or more of the preceding claims, **characterized by** the fact that said tubular element (2) comprises drainage means (14) adapted to promote the drainage of fluids present in said passage channel (3) outside the latter.

6. Device (1) according to claim 5, **characterized by** the fact that:
- said tubular element (2) has a free end (10) arranged, in use, within the patient's intestine (I), said first section (2a) being located between said obstructing means (6) and said free end (10); and by the fact that
- said drainage means (14) comprise at least one drainage hole (14) passable through by said fluids to be drained and cut out on at least one of either said first section (2a) or said free end (10).

7. Device (1) according to claim 6, **characterized by** the fact that said drainage hole (14) is cut out on said first section (2a).

8. Device (1) according to claim 7, **characterized by** the fact that said drainage hole (14) is located between said free end (10) and said inlet openings (4).

9. Device (1) according to one or more of the preceding claims, **characterized by** the fact that it comprises stiffening means (15) adapted to counteract the bending of said tubular element (2).

10. Device (1) according to claim 9, **characterized by** the fact that said stiffening means (15) comprise:
- at least one containment channel (15a) cut out longitudinally through said tubular element (2); and
- at least one stiffening element (15b) inserted within said containment channel (15a).

11. Device (1) according to claim 10, **characterized by** the fact that said containment channel (15a) and said stiffening element (15b) have a longitudinal extension substantially coinciding with that of said tubular element (2).

12. Device (1) according to claim 10 or 11, **characterized by** the fact that said stiffening element (15b) has filiform conformation and is made at least partly of a metallic material.

13. Device (1) according to claim 12, **characterized by** the fact that said stiffening element (15b) has thickness of less than 1 mm.

14. Device (1) according to one or more of the preceding claims, **characterized by** the fact that it comprises retaining means (16) associated with said sheath member (11) at a predefined distance from said obstructing means (6) and intended, in use, to be placed in abutment against the patient's body.

15. Device (1) according to one or more of the preceding claims, **characterized by** the fact that said obstructing means (6) comprise a plurality of elastically deformable tensioning elements (8) associated with said at least a first section (2a) to prevent the folding thereof and locked together where their respective ends are located.

## Patentansprüche

1. Medizinisches Gerät (1) zur Durchführung von Ileostomien und/oder Jejunostomien, umfassend
- mindestens ein rohrförmiges Element (2) mit mindestens einem Durchgangskanal (3), der mit einer Vielzahl von Einlassöffnungen (4) für Fäkalien versehen ist, in den Darm (I) eines Patienten einführbar ist und mindestens eine Auslassöffnung (5) für Fäkalien aufweist, die außerhalb des Darms (I) positionierbar ist, wobei das rohrförmige Element (2) mindestens einen ersten Abschnitt (2a), an dem die Einlassöffnungen (4) ausgebildet sind, und mindestens einen zweiten Abschnitt (2b), an dem die Auslassöffnung (5) ausgebildet ist, aufweist;
- Absperrmittel (6) für mindestens einen Bereich (Z) des Darms (I), die mit dem rohrförmigen Element (2) verbunden sind, die in den Darm (I) einführbar und zwischen einer verengten Konfiguration und einer erweiterten Konfiguration bewegbar sind, um deren Einführen in den Darm (I) zu ermöglichen bzw. den Durchgang von Fäkalien durch den Bereich (Z) zu verhindern, wobei die Absperrmittel (6) zwischen dem ersten Abschnitt (2a) und dem zweiten Abschnitt (2b) angeordnet sind;
**dadurch gekennzeichnet, dass** es mindestens ein Umhüllungselement (11) umfasst, das dem rohrförmigen Element (2) zugeordnet ist, an den Absperrmitteln (6) anliegt und ausgebildet ist, dem entsprechenden Gleiten zwischen den Absperrmitteln (6) und dem rohrförmigen Element (2) entgegenzuwirken.

2. Gerät (1) nach Anspruch 1, **dadurch gekennzeichnet, dass** die Absperrmittel (6) mindestens einen Kontaktabschnitt (12) umfassen, der im Einsatz an dem Umhüllungselement (11) anliegt, wobei der Kontaktabschnitt (12) und das Umhüllungselement (11) an der Stelle, an der sich ihre Kontaktfläche befindet, im Wesentlichen übereinstimmende Querabmessungen aufweisen, um eine nahtlose äußere Verbindungsfläche (13) zu bilden.

3. Gerät (1) nach einem oder mehreren der vorhergehenden Ansprüche, **dadurch gekennzeichnet, dass** das Umhüllungselement (11) zumindest teilweise aus mindestens einem Kunststoffmaterial besteht.

4. Gerät (1) nach Anspruch 3, **dadurch gekennzeichnet, dass** das Umhüllungselement (11) zumindest teilweise aus Silikon besteht.

5. Gerät (1) nach einem oder mehreren der vorstehenden Ansprüche, **dadurch gekennzeichnet, dass** das rohrförmige Element (2) Drainagemittel (14) umfasst, die ausgebildet sind, die Ableitung von in dem Durchgangskanal (3) vorhandenen Flüssigkeiten aus diesem heraus zu fördern.

6. Gerät (1) nach Anspruch 5, **dadurch gekennzeichnet, dass**:
- das rohrförmige Element (2) ein freies Ende (10) aufweist, das im Einsatz innerhalb des Darms (I) des Patienten angeordnet ist, wobei sich der erste Abschnitt (2a) zwischen den Absperrmitteln (6) und dem freien Ende (10) befindet; und dadurch, dass
- die Drainagemittel (14) mindestens eine Ableitöffnung (14) umfassen, die von den abzuleitenden Flüssigkeiten durchströmt werden kann und in mindestens einem der beiden Abschnitte - dem ersten Abschnitt (2a) oder dem freien Ende (10) - ausgebildet ist.

7. Gerät (1) nach Anspruch 6, **dadurch gekennzeichnet, dass** die Ableitöffnung (14) in dem ersten Abschnitt (2a) ausgebildet ist.

8. Gerät (1) nach Anspruch 7, **dadurch gekennzeichnet, dass** sich die Ableitöffnung (14) zwischen dem freien Ende (10) und den Einlassöffnungen (4) befindet.

9. Gerät (1) nach einem oder mehreren der vorhergehenden Ansprüche, **dadurch gekennzeichnet, dass** es Versteifungsmittel (15) umfasst, die ausgebildet sind, der Verbiegung des rohrförmigen Elements (2) entgegenzuwirken.

10. Gerät (1) nach Anspruch 9, **dadurch gekennzeichnet, dass** die Versteifungsmittel (15) umfassen:
- mindestens einen in Längsrichtung durch das rohrförmige Element (2) ausgeschnittenen Aufnahmekanal (15a); und
- mindestens ein in den Aufnahmekanal (15a) eingesetztes Versteifungselement (15b).

11. Gerät (1) nach Anspruch 10, **dadurch gekennzeichnet, dass** der Aufnahmekanal (15a) und das Versteifungselement (15b) eine Längserstreckung aufweisen, die im Wesentlichen mit der des rohrförmigen Elements (2) übereinstimmt.

12. Gerät (1) nach Anspruch 10 oder 11, **dadurch gekennzeichnet, dass** das Versteifungselement (15b) eine fadenförmige Gestalt aufweist und zumindest teilweise aus einem metallischen Werkstoff besteht.

13. Gerät (1) nach Anspruch 12, **dadurch gekennzeichnet, dass** das Versteifungselement (15b) eine Dicke von weniger als 1 mm aufweist.

14. Gerät (1) nach einem oder mehreren der vorhergehenden Ansprüche, **dadurch gekennzeichnet, dass** es Haltemittel (16) umfasst, die dem Umhüllungselement (11) in einem vordefinierten Abstand zu den Absperrmitteln (6) zugeordnet sind und ausgebildet sind, im Einsatz in Anlage gegen den Körper des Patienten gebracht zu werden.

15. Gerät (1) nach einem oder mehreren der vorhergehenden Ansprüche, **dadurch gekennzeichnet, dass** die Absperrmittel (6) eine Vielzahl von elastisch verformbaren Spannelementen (8) umfassen, die mit dem zumindest einen ersten Abschnitt (2a) verbunden sind, um dessen Faltenbildung zu verhindern, und an den Stellen, an denen sich ihre jeweiligen Enden befinden, miteinander verriegelt sind.

## Revendications

1. Dispositif médical (1) pour la réalisation d'iléostomies et/ou de jéjunostomies, comprenant :
- au moins un élément tubulaire (2) comprenant au moins un canal de passage (3) comportant une pluralité d'ouvertures d'entrée (4) de selles, pouvant être introduit dans l'intestin (I) d'un patient, et au moins une ouverture de sortie (5) de selles, pouvant être positionnée à l'extérieur de l'intestin (I), ledit élément tubulaire (2) comprenant au moins une première section (2a), sur laquelle sont définies lesdites ouvertures d'entrée (4), et au moins une seconde section (2b), sur laquelle est définie ladite ouverture de sortie (5) ;
- des moyens d'obstruction (6) d'au moins une zone (Z) de l'intestin (I) associée audit élément tubulaire (2), pouvant être introduits dans l'intestin (I) et mobiles entre une configuration rétrécie et une configuration élargie pour respectivement permettre leur introduction dans l'intestin (I) et empêcher le passage de selles à travers ladite zone (Z), lesdits moyens d'obstruction (6) étant situés entre ladite première section (2a) et ladite seconde section (2b) ;
**caractérisé par le fait qu'**il comprend au moins un élément gaine (11) associé audit élément tubulaire (2), disposé en butée contre lesdits moyens d'obstruction (6) et apte à contrer le glissement correspondant entre lesdits moyens d'obstruction (6) et ledit élément tubulaire (2).

2. Dispositif (1) selon la revendication 1, **caractérisé par le fait que** lesdits moyens d'obstruction (6) comprennent au moins une partie de contact (12) venant en butée, lors de l'utilisation, contre ledit élément gaine (11), ladite partie de contact (12) et ledit élément gaine (11) présentant, à l'endroit où se situe leur zone de contact, des dimensions transversales coïncidant sensiblement pour définir une surface de jonction extérieure sans soudure (13).

3. Dispositif (1) selon l'une ou plusieurs des revendications précédentes, **caractérisé par le fait que** ledit élément gaine (11) est constitué, au moins en partie, d'au moins une matière plastique.

4. Dispositif (1) selon la revendication 3, **caractérisé par le fait que** ledit élément gaine (11) est constitué, au moins en partie, de silicone.

5. Dispositif (1) selon l'une ou plusieurs des revendications précédentes, **caractérisé par le fait que** ledit élément tubulaire (2) comprend des moyens de drainage (14) aptes à favoriser le drainage de fluides présents dans ledit canal de passage (3) vers l'extérieur de ce dernier.

6. Dispositif (1) selon la revendication 5, **caractérisé par le fait que** :
- ledit élément tubulaire (2) a une extrémité libre (10) disposée, lors de l'utilisation, à l'intérieur de l'intestin (I) du patient, ladite première section (2a) étant située entre lesdits moyens d'obstruction (6) et ladite extrémité libre (10) ; et **par le fait que** :
- lesdits moyens de drainage (14) comprennent au moins un trou de drainage (14) pouvant être traversé par lesdits fluides à drainer et découpé sur au moins l'une de ladite première section (2a) ou de ladite extrémité libre (10).

7. Dispositif (1) selon la revendication 6, **caractérisé par le fait que** ledit trou de drainage (14) est découpé sur ladite première section (2a).

8. Dispositif (1) selon la revendication 7, **caractérisé par le fait que** ledit trou de drainage (14) est situé entre ladite extrémité libre (10) et lesdites ouvertures d'entrée (4).

9. Dispositif (1) selon l'une ou plusieurs des revendications précédentes, **caractérisé par le fait qu'**il comprend des moyens de rigidification (15) aptes à contrer la flexion dudit élément tubulaire (2).

10. Dispositif (1) selon la revendication 9, **caractérisé par le fait que** lesdits moyens de rigidification (15) comprennent :
- au moins un canal de confinement (15a) découpé longitudinalement à travers ledit élément tubulaire (2) ; et
- au moins un élément de rigidification (15b) introduit à l'intérieur dudit canal de confinement (15a).

11. Dispositif (1) selon la revendication 10, **caractérisé par le fait que** ledit canal de confinement (15a) et ledit élément de rigidification (15b) ont une extension longitudinale coïncidant sensiblement avec celle dudit élément tubulaire (2).

12. Dispositif (1) selon la revendication 10 ou 11, **caractérisé par le fait que** ledit élément de rigidification (15b) a une conformation filiforme et est constitué, au moins en partie, d'un matériau métallique.

13. Dispositif (1) selon la revendication 12, **caractérisé par le fait que** ledit élément de rigidification (15b) a une épaisseur de moins de 1 mm.

14. Dispositif (1) selon l'une ou plusieurs des revendications précédentes, **caractérisé par le fait qu'**il comprend des moyens de retenue (16) associés audit élément gaine (11) à une distance prédéfinie à partir desdits moyens d'obstruction (6) et destinés, lors de l'utilisation, à être placés en butée contre le corps du patient.

15. Dispositif (1) selon l'une ou plusieurs des revendications précédentes, **caractérisé par le fait que** lesdits moyens d'obstruction (6) comprennent une pluralité d'éléments de tension (8) déformables élastiquement, associés à ladite au moins une première section (2a) pour empêcher son repliement et verrouillés ensemble là où leurs extrémités respectives sont situées.
